# EUROPÄISCHE PATENTSCHRIFT

(11) **EP 0 529 600 B1**
(45) Veröffentlichungstag und Bekanntmachung des Hinweises auf die Patenterteilung: **06.11.1996**
(21) Anmeldenummer: 92114527.2
(22) Anmeldetag: 26.08.1992
(51) Int. Cl.: C07D 277/82

(54) **Verfahren zur Herstellung von 2-Aminobenzthiazolen**
Process for the preparation of 2-aminobenzothiazoles
Procédé de préparation de 2-aminobenzothiazoles

(30) Priorität: 30.08.1991 DE 4128872
(43) Veröffentlichungstag der Anmeldung: 03.03.1993
(73) Patentinhaber: HOECHST AKTIENGESELLSCHAFT, 65926 Frankfurt am Main (DE)
(72) Erfinder: Dapperheld, Steffen, Dr., W-6238 Hofheim/Taunus (DE); Volk, Heinrich, Dr., W-6368 Bad Vilbel (DE); Peter, Karl, W-6458 Rodenbach (DE)

(56) Entgegenhaltungen:
- EP-A- 0 207 416
- US-A- 4 363 913
- CHEMICAL ABSTRACTS, vol. 96, no. 25, 21. Juni 1982, Columbus, Ohio, US; abstract no. 217832q, Seite 744 ; & JP-A-82 009 774

## Beschreibung

2-Aminobenzthiazole sind wichtige Zwischenprodukte zur Herstellung von Farbstoffen und Pflanzenschutzmitteln.

Es ist bekannt, 2-Aminobenzthiazole durch Cyclisierung entsprechender Arylthioharnstoffe in mindestens 85 %iger Schwefelsäure in Gegenwart katalytischer Mengen Brom, Bromwasserstoff, Natrium-, Kalium- oder Ammoniumbromid bei Temperaturen von 2 bis 120°C herzustellen (Japanische Patentanmeldung Sho 57-9774; US-PS 4 363 913). Die Reaktion verläuft jedoch nicht einheitlich, so daß durch Nebenreaktionen (Sulfatierung, Bromierung, Hydrolyse des Phenylthioharnstoffs mit zum Teil nachfolgenden Substitutionen der erstgenannten Art) qualitativ nicht befriedigende Produktgemische entstehen, die eine aufwendige Reinigung erfordern und (bei geringen Löslichkeitsunterschieden der Ziel- und Nebenprodukte) schlechte Ausbeuten ergeben.

Diese Verfahren sind jedoch mit dem Nachteil behaftet, daß die eingesetzten Arylthioharnstoffe nur in trockenem Zustand eingesetzt werden. Außerdem sind die Ausbeuten meist unbefriedigend.

Die Trocknung dieser Verbindungen ist zwar ein technisch unproblematischer Prozeß, der aber die Wirtschaftlichkeit des Verfahrens negativ beeinflußt, da die meist sehr toxischen und stark staubenden Produkte in der Praxis nur unter aufwendigen Sicherheitsmaßnahmen zu handhaben sind.

Durch die vorliegende Erfindung wird nunmehr ein Verfahren zur Herstellung von 2-Amino-benzthiazolen zur Verfügung gestellt, bei dem die genannten Nachteile der üblichen Verfahren nicht mehr auftreten.

Es wurde nun gefunden, daß man 2-Amino-benzthiazole der Formel (I) (s. Patentanspruch 1) worin R₁ und R₂ unabhängig voneinander Wasserstoff, Fluor, Chlor, Brom oder Jod, Trifluormethyl oder C₁-C₄-Alkyl-, C₁-C₄-Alkoxy- oder Nitrogruppen bedeuten, in hoher Ausbeute und Reinheit durch Umsetzung von Arylthioharnstoffen der Formel (II) (s. Patentanspruch 1), worin R₁ und R₂ die genannte Bedeutung haben, herstellen kann, wenn man die Umsetzung in 99 bis 100 %iger Schwefelsäure unter kontinuierlicher Zugabe katalytischer Mengen an Brom, Bromwasserstoff oder Bromid in Form wäßriger Lösungen durchführt und wenn man Arylthioharnstoffe der Formel (II) einsetzt, die 1 bis 35 % Wasser enthalten.

Als Bromide kommen beispielsweise Alkalimetallbromide, Ammoniumbromid und Tetraalkylammoniumbromid in Frage, bevorzugt jedoch Natrium-, Kalium- oder Ammoniumbromid.

Bevorzugte Verbindungen der Formel (I) sind:
4-Chlor-, 6-Chlor-, 5,6-Dichlor-, 4-Fluor-, 6-Fluor, 6-Nitro-, 4-Nitro-, 6-Methoxy-, 6-Ethoxy-, 6-Methyl-, 6-Ethyl-, 4-Trifluormethyl-, 6-Trifluormethyl-2-aminobenzthiazol.

Von den Verbindungen der Formel (II) sind 2-Chlor-, 4-Chlor-, 3,4-Dichlor-, 2-Fluor-4-Fluor-, 4-Trifluormethyl-, 2-Nitro-, 4-Nitro-, 4-Methoxy-, 4-Ethoxy-, 4-Methyl-, 2-Methyl-, 2-Ethyl-, 4-Ethylphenylthioharnstoff besonders bevorzugt. Diese Verbindungen enthalten bevorzugt 2 bis 25 %, insbesondere 3 bis 20 %, Wasser.

Die Ausgangsverbindungen (II) lassen sich nach üblichen Methoden herstellen, wie sie beispielsweise in Houben-Weyl, Methoden der Organischen Chemie, Bd. 9 (1955), S. 888 beschrieben sind.

Die Verbindungen der Formel (II) werden in 99 bis 100 %iger Schwefelsäure, vorzugsweise in 100 %iger Schwefelsäure, gelöst. Die Menge der Schwefelsäure beträgt im allgemeinen das mindestens 1,5-fache der Menge an eingesetztem Arylthioharnstoff, bevorzugt ist der Einsatz der 1,9 bis 5-fachen, insbesondere der 2- bis 3,4-fachen Menge an Schwefelsäure. Die Verwendung größerer Mengen an Schwefelsäure ist möglich, verringert aber die Wirtschaftlichkeit des Verfahrens.

Bezogen auf die Gesamtmenge an eingesetztem Arylthioharnstoff und Schwefelsäure ist im allgemeinen ein Wassergehalt von 2 bis 20 Gew.-%, vorzugsweise von 2,5 bis 15 Gew.-%, insbesondere von 3 bis 8 Gew.-%, von Vorteil.

Das erfindungsgemäße Verfahren wird im allgemeinen bei Temperaturen zwischen 10 und 130°C, bevorzugt zwischen 20 und 100°C, durchgeführt. Die Reaktionstemperatur hängt im wesentlichen von der Art der Substituenten R¹ und R² ab, da der Einsatz von Verbindungen mit elektronegativen Substituenten in der Regel höhere Temperaturen erfordert als ein solcher von Verbindungen mit elektropositiven Substituenten.

Das Bromid, das in katalytischen Mengen der Reaktionslösung zugesetzt wird, kann als Feststoff oder in Form einer wäßrigen Lösung, als wäßrige Lösung von Bromwasserstoff oder als elementares Brom kontinuierlich zudosiert werden. Bevorzugt ist die kontinuierliche Zugabe wäßriger Lösungen von Ammonium-, Natrium- oder Kaliumbromid bzw. Bromwasserstoff, insbesondere die kontinuierliche Zugabe von Ammoniumbromid-Lösung. Die wäßrigen Lösungen dieser Substanzen werden möglichst konzentriert eingesetzt, um den Gehalt der eingesetzten 99 bis 100 %igen Schwefelsäure möglichst wenig zu verringern. Bevorzugt ist der Einsatz gesättigter Lösungen. Die Menge des zugesetzten Bromids liegt im allgemeinen zwischen 0,01 und 10 Mol-%, bevorzugt zwischen 0,1 und 5 Mol.-%, bezogen auf die eingesetzte Menge an Arylthioharnstoff.

Das entstandene Abgas (SO₂) wird in einer mit Alkalilauge betriebenen Gaswäsche zu wiederverwendbarer Bisulfitlauge umgesetzt.

Der Fortgang der Reaktion ist durch analytische Verfahren, wie Dünnschicht-, Gas- oder Flüssigkeitschromatographie, leicht zu verfolgen.

Nach dem vollständigen Umsatz der Ausgangsverbindungen wird das Reaktionsgemisch mit Wasser verdünnt und das gebildete 3-Aminobenzthiazol der Formel (I) in Form seines Sulfates durch Abkühlen ausgeschieden oder als freie Base durch Zusatz von Alkalilauge oder wäßriger Ammoniaklösung ausgefällt und durch Filtration isoliert.

### Beispiele

1. 117,6 g 2-Chlorphenylthioharnstoff mit einem Trockengehalt von 85 % wurden bei 20 bis 25°C innerhalb einer halben Stunde in 324 g H₂SO₄ (100 %) gelöst. Das Gewichtsverhältnis von Schwefelsäure zu 2-Chlorphenylthioharnstoff (100 %) betrug 3,24 und der Wassergehalt betrug, bezogen auf die Summe von 2-Chlorphenylthioharnstoff und H₂SO₄, 3,98 Gew.-%. Dann wurde auf 70°C erwärmt, und innerhalb von 4 Stunden 12 ml einer 40 %igen NH₄Br-Lösung zugetropft. Die resultierende Lösung wurde in 400 ml Wasser eingerührt und eine Stunde bei 70°C gerührt. Es wurde auf 20°C abgekühlt und abgesaugt. Der Filterrückstand wurde in eine Lösung aus 1150 ml Wasser und 50 g NaOH eingetragen und eine Stunde bei 70°C gerührt. Dann wurde abgesaugt und mit Wasser sulfatfrei gewaschen und getrocknet.
   - Ausbeute:: 94,3 g 4-Chlor-2-aminobenzthiazol (96,7 %), Schmelzpunkt: 202-203°C.
2.-5. Es wurde wie im Beispiel 1 verfahren, jedoch wurde der 2-Chlorphenylthioharnstoff durch die in der Tabelle 1 angegebenen Verbindungen ersetzt. 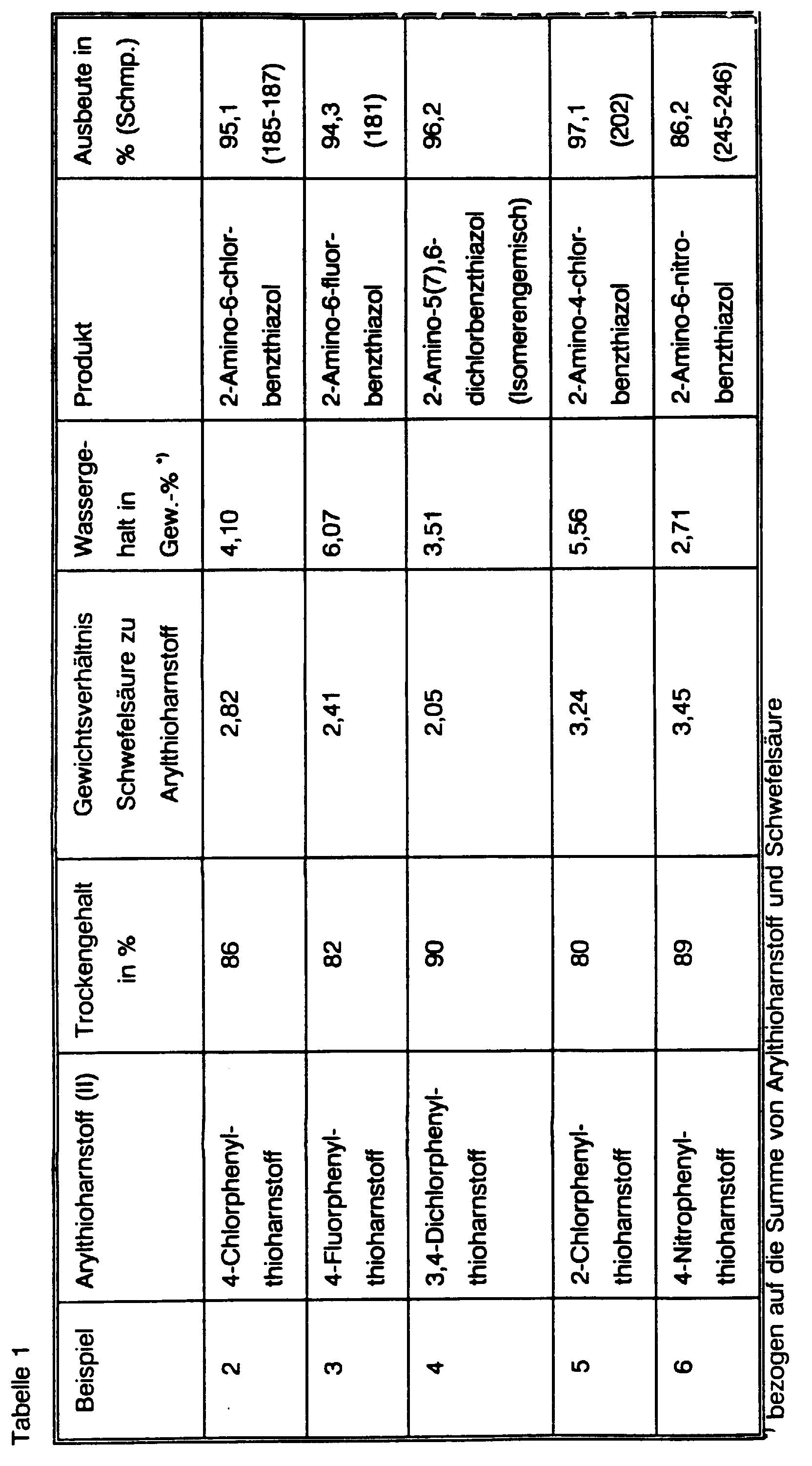
7. 100 g (0,5 Mol) 4-Methoxyphenylthioharnstoff (Trockengehalt 91 %) wurden innerhalb einer halben Stunde bei 20 bis 25°C portionsweise in 290 g H₂SO₄ (100 %) gelöst. Das Gewichtsverhältnis von Schwefelsäure zu 4-Methoxyphenylthioharnstoff (100 %) betrug 3,19 und der Wassergehalt betrug, bezogen auf die Summe von 4-Methoxyphenylthioharnstoff und Schwefelsäure, 2,47 Gew.-%. Dann wurden bei 25 bis 30°C innerhalb von 3 Stunden 30 g einer 40 %igen NH₄Br-Lösung kontinuierlich zudosiert. Anschließend wurde die Lösung in 1200 g Wasser gegeben, bei 50°C klarfiltriert und mit 430 ml einer 33 %igen NaOH-Lösung (143 g NaOH) auf pH = 8 eingestellt. Es wurde eine halbe Stunde bei 40°C nachgerührt und abgesaugt. Mit 1500 g Wasser wurde sulfatfrei gewaschen und anschließend getrocknet.
   - Ausbeute:: 85 g 2-Amino-6-methoxybenzthiazol (94,4 %), Schmelzpunkt: 160-161°C.
8. 108,3 g 4-Chlorphenylthioharnstoff (Trockengehalt 86,1 %) wurden bei 20°C in 320 g H₂SO₄ (100 %) gelöst. Das Gewichtsverhältnis von Schwefelsäure zu 4-Chlorphenylthioharnstoff betrug 3,4 und der Wassergehalt betrug.., bezogen auf die Summe von 4-Chlorphenylthioharnstoff und Schwefelsäure, 3,51 Gew.-%. Dann wurden innerhalb von 3 Stunden kontinuierlich 6 g HBr-Lösung (48 %ig) zugesetzt. Die Temperatur wurde für 1,5 Stunden bei 45 bis 50°C und dann für 6 Stunden bei 65 bis 70°C gehalten. Die Lösung wurde auf 20°C gekühlt und es wurden 250 ml Methanol zugesetzt. Die Temperatur stieg auf ca. 70°C an. Dann wurde auf 20°C gekühlt und abgesaugt. Der Filterrückstand wurde dreimal mit je 150 ml Aceton gewaschen und an der Luft getrocknet.
   - Ausbeute:: 122,6 g 2-Amino-4-chlorbenzthiazol-Sulfat (87,7 %).

### Vergleichsbeispiele

1. 100 g (0,53 Mol) 2-Chlorphenylthioharnstoff (100 %) wurden bei 20 bis 25°C innerhalb einer halben Stunde in 324 g H₂SO₄ (95 %) gelöst. Das Gewichtsverhältnis von Schwefelsäure (100 %) zu 2-Chlorphenylthioharnstoff betrug 3,08 und der Wassergehalt betrug, bezogen auf die Summe von 2-Chlorphenylthioharnstoff und Schwefelsäure, 3,8 Gew.-%. Dann wurde auf 70°C erwärmt und innerhalb von 4 Stunden 12 ml einer 40 %igen NH₄Br-Lösung zugegeben. Die resultierende Lösung wurde in 400 ml Wasser eingerührt und eine Stunde bei 70°C gerührt. Es wurde auf 20°C abgekühlt und abgesaugt. Der Filterrückstand wurde in eine Lösung aus 1150 ml Wasser und 50 g NaOH eingetragen und eine Stunde bei 70°C gerührt. Dann wurde abgesaugt und mit Wasser sulfatfrei gewaschen.
   - Ausbeute:: 79 g 4-Chlor-2-aminobenzthiazol (81 %), Schmelzpunkt: 200-201°C.
2. Bei diesem Beispiel handelt es sich um eine direkte Nacharbeitung des Beispiels 6 aus der US-PS 4,363,913 und ist direkt mit dem erfindungsgemäßen Beispiel 8 zu vergleichen.
   93,3 g 4-Chlorphenylthioharnstoff wurden bei 20°C in 150 ml H₂SO₄ (98 %) gelöst. Das Gewichtsverhältnis von Schwefelsäure zu 4-Chlorphenylthioharnstoff betrug 3,0 und der Wassergehalt betrug, bezogen auf die Summe von 4-Chlorphenylthioharnstoff und Schwefelsäure, 1,5 Gew.-%. Dann wurden innerhalb von 3 Stunden im Abstand von 30 Minuten je 1 g HBr-Lösung (48 %ig) zugesetzt. Die Temperatur wurde für 1,5 Stunden bei 45 bis 50°C und dann für 6 Stunden bei 65 bis 70°C gehalten.
   Die Lösung wurde auf 20°C gekühlt und es wurden 250 ml Methanol zugesetzt. Die Temperatur stieg auf ca. 70°C an. Dann wurde auf 20°C gekühlt und abgesaugt. Der Filterrückstand wurde dreimal mit je 150 ml Aceton gewaschen und an der Luft getrocknet.
   - Ausbeute:: 99,7 g 2-Amino-4-chlorbenzthiazol-Sulfat (Wiederholungsversuch: 101,4 g), Durchschnittsausbeute: 100,55 g (71,9 %).
3. Es wurde wie in Vergleichsbeispiel 2 verfahren, wobei jedoch 108,3 g 4-Chlorphenylthioharnstoff mit einem Trockengehalt von 86,1 % eingesetzt wurden. Das Gewichtsverhältnis von Schwefelsäure zu 4-Chlorphenylthioharnstoff betrug 3,4 und der Wassergehalt betrug, bezogen auf die Summe von 4-Chlorphenylthioharnstoff und Schwefelsäure, 5,37 Gew.-%.
   - Ausbeute:: 35,5 g 2-Amino-4-chlorbenzthiazol-Sulfat (25,4 %).

## Patentansprüche

1. Verfahren zur Herstellung von 2-Aminobenzthiazolen der Formel (I) 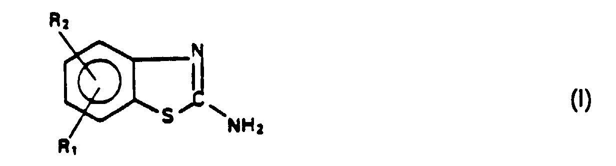 worin R₁ und R₂ unabhängig voneinander Wasserstoff, Fluor, Chlor, Brom oder Jod, Trifluormethyl oder C₁-C₄-Alkyl-, C₁-C₄-Alkoxy- oder Nitrogruppen bedeuten, durch Umsetzung von Arylthioharnstoffen der Formel (II) 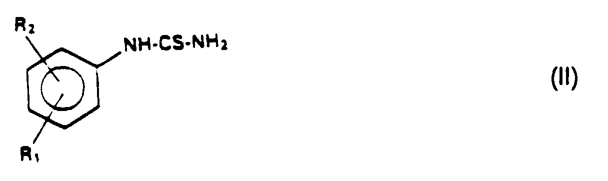 worin R₁ und R₂ die genannte Bedeutung haben, dadurch gekennzeichnet, daß man die Umsetzung in 99 bis 100 %iger Schwefelsäure unter kontinuierlicher Zugabe katalytischer Mengen an Brom, Bromwasserstoff oder Bromid in Form wäßriger Lösungen durchführt und daß man Arylthioharnstoffe der Formel (II) einsetzt, die 1 bis 35 % Wasser enthalten.

2. Verfahren nach Anspruch 1, dadurch gekennzeichnet, daß die Reaktion in 100 %iger Schwefelsäure durchgeführt wird.

3. Verfahren nach Anspruch 1 oder 2, dadurch gekennzeichnet, daß als Bromid Natrium-, Kalium- oder Ammoniumbromid eingesetzt wird.

4. Verfahren nach einem oder mehreren der Ansprüche 1 bis 3, dadurch gekennzeichnet, daß es sich bei den Verbindungen der Formel (I) um 4-Chlor-, 6-Chlor-, 5,6-Dichlor-, 4-Fluor-, 6-Fluor, 6-Nitro-, 4-Nitro-, 6-Methoxy-, 6-Ethoxy-, 6-Methyl-, 6-Ethyl-, 4-Trifluormethyl-, 6-Trifluormethyl-2-aminobenzthiazol handelt.

5. Verfahren nach einem oder mehreren der Ansprüche 1 bis 4, dadurch gekennzeichnet, daß als Arylthioharnstoffe der Formel (II) 2-Chlor-, 4-Chlor-, 3,4-Dichlor-, 2-Fluor-, 4-Fluor-, 4-Trifluormethyl-, 2-Nitro-, 4-Nitro-, 4-Methoxy-, 4-Ethoxy-, 4-Methyl-, 2-Methyl-, 2-Ethyl- oder 4-Ethylphenylthioharnstoff eingesetzt werden.

6. Verfahren nach einem oder mehreren der Ansprüche 1 bis 5, dadurch gekennzeichnet, daß die Arylthioharnstoffe 2 bis 25 %, bevorzugt 3 bis 20 %, Wasser enthalten.

7. Verfahren nach einem oder mehreren der Ansprüche 1 bis 6, dadurch gekennzeichnet, daß man die Umsetzung in der mindestens 1,5-fachen, vorzugsweise der 1,9- bis 5-fachen, besonders bevorzugt der 2- bis 3,4-fachen, Gewichtsmenge an Schwefelsäure, bezogen auf den Arylthioharnstoff, durchführt.

8. Verfahren nach einem oder mehreren der Ansprüche 1 bis 7, dadurch gekennzeichnet, daß die Ausgangsprodukte, bezogen auf die Summe von Arylthioharnstoff und Schwefelsäure, 2 bis 20 Gew.-%, vorzugsweise 2,5 bis 15 Gew.-%, besonders bevorzugt 3 bis 8 Gew.-%, Wasser enthalten.

9. Verfahren nach einem oder mehreren der Ansprüche 1 bis 8, dadurch gekennzeichnet, daß das Bromid in einer Menge von 0,01 bis 10 Mol-%, vorzugsweise von 0,1 bis 5 Mol-%, bezogen auf die eingesetzte Menge an Arylthioharnstoff eingesetzt wird.

10. Verfahren nach Anspruch 9, dadurch gekennzeichnet, daß das Bromid in Form einer gesättigten Lösung eingesetzt wird.

11. Verfahren nach einem oder mehreren der Ansprüche 1 bis 10, dadurch gekennzeichnet, daß die Reaktion bei einer Temperatur von 10 bis 130°C, vorzugsweise von 20 bis 100°C durchgeführt wird.

## Claims

1. A process for the preparation of a 2-aminobenzothiazole of the formula (I) 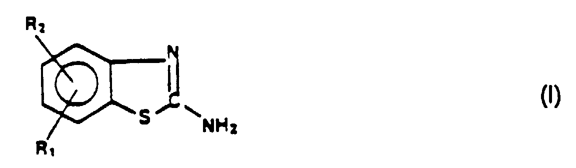 in which R₁ and R₂ independently of each other are hydrogen, fluorine, chlorine, bromine or iodine, trifluoromethyl or C₁-C₄-alkyl, C₁-C₄-alkoxy or nitro groups, by conversion of an arylthiourea of the formula (II) 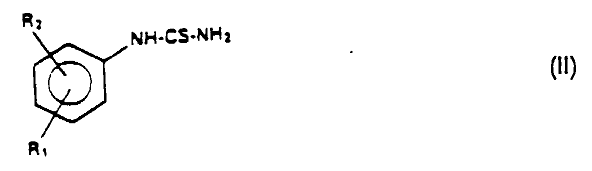 in which R₁ and R₂ have the meaning mentioned, which comprises carrying out the conversion in 99 to 100% strength sulfuric acid with continuous addition of catalytic amounts of bromine, hydrogen bromide or bromide in the form of aqueous solutions and using an arylthiourea of the formula (II) which contains 1 to 35% water.

2. The process as claimed in claim 1, wherein the reaction is carried out in 100% strength sulfuric acid.

3. The process as claimed in claim 1 or 2, wherein the bromide used is sodium bromide, potassium bromide or ammonium bromide.

4. The process as claimed in one or more of claims 1 to 3, wherein the compound of the formula (I) is 4-chloro-, 6-chloro-, 5,6-dichloro-, 4-fluoro-, 6-fluoro-, 6-nitro-, 4-nitro-, 6-methoxy-, 6-ethoxy-, 6-methyl-, 6-ethyl-, 4-trifluoromethyl- or 6-trifluoromethyl-2-aminobenzothiazole.

5. The process as claimed in one or more of claims 1 to 4, wherein the arylthiourea of the formula (II) used is 2-chloro-, 4-chloro-, 3,4-dichloro-, 2-fluoro-, 4-fluoro-, 4-trifluoromethyl-, 2-nitro-, 4-nitro-, 4-methoxy-, 4-ethoxy-, 4-methyl-, 2-methyl, 2-ethyl- or 4-ethylphenylthiourea.

6. The process as claimed in one or more of claims 1 to 5, wherein the arylthiourea contains 2 to 25%, preferably 3 to 20%, water.

7. The process as claimed in one or more of claims 1 to 6, wherein the conversion is carried out with at least 1.5-fold, preferably 1.9 to 5-fold, particularly preferably 2 to 3.4-fold amounts by weight of sulfuric acid, relative to the arylthiourea.

8. The process as claimed in one or more of claims 1 to 7, wherein the starting compounds, relative to the sum of arylthiourea and sulfuric acid, contain 2 to 20% by weight, preferably 2.5 to 15% by weight, particularly preferably 3 to 8% by weight, of water.

9. The process as claimed in one or more of claims 1 to 8, wherein the bromide is used in an amount of 0.01 to 10 mol %, preferably 0.1 to 5 mol %, relative to the amount of arylthiourea used.

10. The process as claimed in claim 9, wherein the bromide is used in the form of a saturated solution.

11. The process as claimed in one or more of claims 1 to 10, wherein the reaction is carried out at a temperature of 10 to 130°C, preferably 20 to 100°C.

## Revendications

1. Procédé pour la préparation de 2-aminobenzothiazoles de formule I 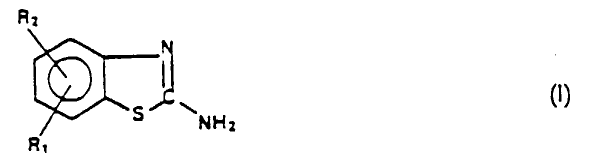 dans laquelle R₁ et R₂, indépendamment l'un de l'autre, représentent l'hydrogène, le fluor, le chlore, le brome ou l'iode, trifluorométhyle ou des groupes alkyles en C₁-C₄, alcoxy en C₁-C₄ ou nitro, par réaction des alkylthiourées de formule II 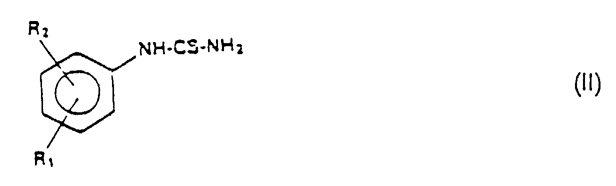 dans laquelle R₁ et R₂ ont la signification précitée, caractérisé en ce qu'on met en oevre la réaction en milieu d'acide sulfurique à 99 à 100%, en ajoutant en continu des quantités catalytiques de brome, de bromure d'hydrogène ou de bromure sous forme de solution aqueuse et en ce qu'on utilise des arylthiourées de formule II qui contiennent de 1 à 35% d'eau.

2. Procédé selon la revendication 1, caractérisé en ce qu'on met en oeuvre la réaction en milieu d'acide sulfurique à 100%.

3. Procédé selon la revendication 1 ou 2, caractérisé en ce qu'on utilise en tant que bromure du bromure de sodium, de potassium ou d'ammonium.

4. Procédé selon une ou plusieurs des revendications 1 ou 3, caractérisé en ce que dans le cas des composés de formule I, il s'agit de : 4-chloro, 6-chloro, 5,6-dichloro, 4-fluoro, 6-fluoro, 6-nitro, 4-nitro, 6-méthoxy, 6-éthoxy, 6-méthyle, 6-éthyle, 4-trifluorométhyle, 6-trifluorométhyl-2-aminobenzothiazole.

5. Procédé selon une ou plusieurs des revendications 1 ou 4, caractérisé en ce que dans le cas des arylthiourées de formule II on utilise des 2-chloro, 4-chloro, 3,4-dichloro, 2-fluoro, 4-fluoro, 4-trifluorométhyle, 2-nitro, 4-nitro, 4-méthoxy, 4-éthoxy, 4-méthyle, 2-méthyle, 2-éthyle ou 4-éthylphénylthiourée.

6. Procédé selon une ou plusieurs des revendications 1 ou 5, caractérisé en ce que les arylthiourées contiennent de 2 à 25%, de préférence de 3 à 20% d'eau.

7. Procédé selon une ou plusieurs des revendications 1 ou 6, caractérisé en ce qu'on met en oeuvre la réaction dans une quantité d'acide sulfurique au moins 1,5 fois, de préférence 1,9 à 5 fois, de manière particulièrement préférée de 2 à 3,5 fois par rapport la quantité d'arylthiourée.

8. Procédé selon une ou plusieurs des revendications 1 ou 7, caractérisé en ce que les produits de départ par rapport à la somme d'arylthiourée et d'acide sulfurique contient de 2 à 20% en poids, de préférence de 2,5 à 15% en poids, de manière particulièrement préférée de 3 à 8% en poids d'eau.

9. Procédé selon une ou plusieurs des revendications 1 ou 8, caractérisé en ce qu'on utilise le bromure dans une quantité de 0,01 à 10% en moles, de préférence de 0,1 à 5% en moles par rapport à la quantité d'arylthiourée utilisée.

10. Procédé selon la revendication 9, caractérisé en ce qu'on utilise le bromure sous forme d'une solution saturée.

11. Procédé selon une ou plusieurs des revendications 1 à 10, caractérisé en ce qu'on met en oeuvre la réaction à une température de 10 à 130°C, de préférence de 20 à 100°C.
